# EUROPEAN PATENT SPECIFICATION

(11) **EP 3 884 855 B1**
(45) Date of publication and mention of the grant of the patent: **24.01.2024**
(21) Application number: 20217975.0
(22) Date of filing: 31.12.2020
(51) Int. Cl.: A61B 5/021, G16H 50/50, A61B 5/022, A61B 8/06, A61B 5/02, A61B 5/107, A61B 5/00

(54) **IMAGE-FREE ULTRASOUND FOR NON-INVASIVE ASSESSMENT OF EARLY VASCULAR HEALTH MARKERS**
BILDFREIER ULTRASCHALL ZUR NICHTINVASIVEN BEURTEILUNG VON FRÜHZEITIGEN MARKERN DER GEFÄSSGESUNDHEIT
ÉCHOGRAPHIE SANS IMAGE POUR L'ÉVALUATION NON INVASIVE DES MARQUEURS PRÉCOCES DE LA SANTÉ VASCULAIRE

(30) Priority: 26.03.2020 IN 202041013190
(43) Date of publication of application: 29.09.2021
(73) Proprietor: Healthcare Technology Innovation Centre, Chennai, Tamil Nadu 600113 (IN); Indian Institute of Technology Madras (IIT Madras), Chennai 600036 (IN)
(72) Inventor: JAYARAJ, JOSEPH, 600113 Chennai (IN); NABEEL PILAPARAMBIL, MASHOOD, 600113 Chennai (IN); MALAY ILESH, SHAH, 600113 Chennai (IN); RAJ KIRAN, VANGAPANDU, 600113 Chennai (IN); MOHANASANKAR, SIVAPRAKASAM, 600113 Chennai (IN)
(74) Representative: Studio Torta S.p.A.

(56) References cited:
- EP-A2- 3 157 416
- US-A1- 2005 154 299
- US-A1- 2017 042 431
- RAJ KIRAN V ET AL: "Analytic Phase Based Approach for Arterial Diameter Evaluation Using A-Mode Ultrasound Frames", 2019 IEEE INTERNATIONAL SYMPOSIUM ON MEDICAL MEASUREMENTS AND APPLICATIONS (MEMEA), IEEE, 26 June 2019 (2019-06-26), pages 1-5, XP033597373, DOI: 10.1109/MEMEA.2019.8802185 [retrieved on 2019-08-14]

## Description

### FIELD OF THE INVENTION

The present invention is related to an image-free ultrasound system for non-invasive assessment of early vascular health markers by measurement of arterial dimensions and pulse waves to evaluate the endothelial function, local, and regional vascular stiffness.

### BACKGROUND OF THE INVENTION

Background description includes information that may be useful in understanding the present invention. It is not an admission that any of the information provided herein is prior art or relevant to the presently claimed invention, or that any publication specifically or implicitly referenced is prior art.

Early detection and timely intervention are key aspects in reducing the mortality and morbidity in cardiovascular disease. Currently used markers for detection of at-risk subjects for primary prevention, such as age, family history of the disease, dyslipidemia, diabetes, central obesity etc., provide an indirect indication of the development and progression of the disease that occurs in the blood vessels and the heart. The earliest sign of the diseases can be detected by the changes in the function of the endothelium, which is the innermost lining of the blood vessel which controls the dynamic ability of the blood vessel to increase or decrease the vessel wall stiffness in response to the autonomic regulatory activity of the body. Loss of endothelial function leads to increased stiffness of the vessel wall, which then leads to the increased wall thickness and plaque formation, eventually leading to an adverse cardiovascular and cerebrovascular event like angina, ischemia, heart failure, or stroke.

Endothelial function is assessed non-invasively using an ultrasound imaging system, by performing flow mediated dilation (FMD) at a peripheral artery such as the brachial artery.

Vascular stiffness is another important marker that may be measured locally from a small segment of an artery (for example: carotid artery, femoral artery, brachial artery, etc.) or globally over a long arterial trajectory (for example: carotid-to-femoral segment, carotid-to-brachial segment, etc.), and is proven to have a clinically relevant role in cardiovascular hemodynamics. In the current practice (exploiting the devices in Table 1), the important role of vascular stiffness in risk stratification has been mostly demonstrated by means of the pulse wave velocity (PWV). The measure of PWV obtained from large arterial sections henceforth referred to as regional PWV (or regional stiffness). The current techniques typically measure PWV across the carotid-femoral segment. Such a regional estimate of the carotid-to-femoral PWV is well known as aortic pulse wave velocity (aortic PWV) or aortic stiffness. Evidence from numerous trials on the independent predictive role of aortic PWV has established its prominent position in guidelines and clinical practices. Subsequently, it is considered as the gold-standard estimate of vascular stiffness.

**Table 1. Commercial Devices for Vascular Stiffness Measurement**

| **Device** | **Manufacturer** | **Sensing modality/sensor** |
|---|---|---|
| SphygmoCor | AtCorMedical, Australia | Tonometer and ECG Tonometer and thigh cuff |
| Complior | Alam Medical, France | Piezoelectric transducer |
| PulsePen | DiaTecne, Italy | Tonometer and ECG |
| PulseTrace | Micro Medical, United Kingdom | Doppler Probe and ECG |
| Vicorder | Skidmore Medical, United Kingdom | Neck pad (partial cuff) and bladder-type thigh/arm cuff |
| Arteriograph | TensioMed, Hungary | Bladder-type pressure cuff |
| Mobil-O-Graph | IEM Healthcare, Germany | Bladder-type pressure cuff |
| CAVI-VaSera | Fukuda Denshi, Japan | ECG and Pressure cuffs |
| Omron VP-1000 | Omron Medical, Japan | Arm and ankle pressure cuff |
| Q-KD | Novacor, France | Bladder-type pressure cuff and ECG |
| NIHem | Cardiovascular Engineering Inc., USA | Tonometer and ECG |
| Echo-tracking techniques: Artlab System E-Tracking HDI-lab | Esaote, Italy Aloka, Japan Philips, Netherlands | Doppler probes and ECG, Ultrasound medical imaging, Echo tracking |

Stiffness of any given artery of interest, measured from its small segments is referred to as local stiffness. Several local stiffness indices (enlisted in the following section) are more closely related to the biomechanical characteristics of the artery. They have established correlations with cardiovascular or cerebrovascular diseases and all-cause mortality. Dedicated medical imaging systems (ultrasound, MRI, etc.) are currently used for local stiffness measurement, typically from the aorta or common carotid artery.

Thus, endothelial dysfunction and increased vascular stiffness are early markers of vascular health that also have established predictive value for cardiovascular mortality and morbidity. However, the routine clinical use of such markers is hampered by the lack of easy-to-use technology for reliable non-invasive assessment, at an affordable cost. In view of the above, there are few prior art patents or patent applications that address the aforementioned problem in a variety of methods. United States Patent Application No. 20160095572A1 discloses a system and method for continuous real time measurement of blood pressure in a subject. The system includes a transducer assembly (e.g., having ultrasound array elements) in a cuff applied to the subject's body. The system measures physical characteristics such as geometry, elasticity and strain in a blood vessel as well as other external physical parameters by means of ultrasound images. Computer modeling and signal processing of measured signals are used during inflation and/or deflation of the cuff to iteratively estimate the blood pressure of the subject. Also, United States Patent Application No. 20110125023A1 also discloses an operation of a patient's heart or lungs by transmitting ultrasound energy into the patient's lung, and detecting Doppler shifts of reflected ultrasound induced by moving borders between blood vessels in the lung and air-filled alveoli that surround the blood vessels. Movement of the border is caused by pressure waves in the blood vessels that result in changes in diameter of those blood vessels. European Patent EP 3157416 A2 discloses a method and system for cuff-less blood pressure (BP) measurement of a subject. The method includes measuring, by one or more sensors, a local pulse wave velocity (PWV) and/or blood pulse waveforms of an arterial wall of the subject. Further, the method includes measuring, by an ultrasound transducer, a change in arterial dimensions over a cardiac cycle of the arterial wall of the subject. The arterial dimensions include an arterial distension and an end-diastolic diameter. Furthermore, the method includes measuring, by a controller unit, BP of the subject based on the local PWV and the change in arterial dimensions.

United States Patent Application No. US 2017/042431 A1 discloses methods and apparatus for assessing endothelial function in a mammal. In certain embodiments the methods involve using a cuff to apply pressure to an artery in a subject to determine a plurality of baseline values for a parameter related to endothelial function as a function of applied pressure (Pm); b) applying a stimulus to the subject, and applying external pressure Pm to the artery to determine a plurality of stimulus-effected values for the parameter related to endothelial function as a function of applied pressure (Pm); where the baseline values are determined from measurements made when said mammal is not substantially effected by said stimulus and differences in said baseline values and said stimulus-effected values provide a measure of endothelial function in said mammal. However, these prior art applications do not address the aforementioned need for detection of early markers that indicate vascular stiffness, both local and regional, in routine clinical diagnostic practices.

Therefore, there is a need for a comprehensive measure of vascular stiffness accounting both the local and regional indices since it has strong potential in stratifying risk of future cardiovascular and other life-threatening events. Indeed, there is an overwhelming need for simultaneous measurement of both the local and regional vascular stiffness in routine clinical diagnostic practices. However, existing devices or technologies are not amenable for such combined analyses, especially with provisions for easy-to-use, minimal operator dependency, portability, and field deployability.

### SUMMARY OF THE INVENTION

It is intended that all such features, and advantages be included within this description, be within the scope of the present invention, and be protected by the accompanying claims. The following summary is provided to facilitate an understanding of some of the innovative features unique to the disclosed embodiment and is not intended to be a full description. A full appreciation of the various aspects of the embodiments disclosed herein can be gained by taking the entire specification, claims, drawings, and abstract as a whole.

The present invention discloses an image-free ultrasound system, which can provide a comprehensive evaluation of vascular health by performing the non-invasive assessment of endothelial function, local vascular stiffness at any superficial artery, and regional vascular across an arterial segment.

The system disclosed here consists of a compact, easy-to-use, field-deployable ultrasound device that does not construct any form of ultrasound images and perform a non-invasive assessment of the following measures, namely:
1) Assessment of endothelial function at any specific superficial artery by flow mediated dilation without the need for imaging references
2) Measurement of local vascular stiffness at any specific superficial artery
3) Measurement of regional vascular stiffness
4) Simultaneous and continuous beat-by-beat measurement of both local and regional vascular stiffness indices, without the use of imaging systems nor ECG signals for signal gating
5) Non-invasive assessment of arterial dimensions and their dynamic variations,

The device is equipped with a single-element ultrasound transducer and at least one flow restrictor, powered by a fully-automated instrument. The device is connected to any laptop computer/tablet/mobile phone or operated independently. The device's intelligent measurement system incorporates programs for simultaneous controlling of the hardware modules and sensing/measuring elements, real-time processing of the acquired signals, algorithms for obtaining desired physiological measurements, evaluation of the local and regional vascular stiffness indices, assessment of endothelial dysfunction, and display the results to the user via a custom interface.

In view of the above, an image-free ultrasound system disclosed here addresses the need for simultaneous, continuous and real-time non-invasive assessment of early vascular health markers. The image-free ultrasound system comprises one or more ultrasound transducers, an ultrasound module, one or more flow restrictors, a pulse detection module, and a measurement module. The ultrasound transducers are positioned at one or more arteries, where the ultrasound transducers generate signals based on blood flow and pulse propagation in the arteries. The ultrasound module is in communication with each ultrasound transducer, where the ultrasound module generates characteristic waves based on the generated signals at the ultrasound transducers. The flow restrictors are positioned at the arteries to restrict blood flow in the arteries, where the flow restrictors generate signals based on the blood flow in the arteries. The pulse detection module is in communication with each flow restrictor, where the pulse detection module generates pulse waves based on the generated signals at the flow restrictor. The measurement module is controlled by at least one processor, where the measurement module is in communication with the ultrasound module and the pulse detection module to receive the generated characteristic waves and the pulse waves respectively, and where the early vascular health markers are measured by the measurement module based on the characteristic waves and the pulse waves.

In an embodiment, the early vascular health markers comprise regional stiffness indices, local stiffness indices, and assessment of endothelial function. In an embodiment, the ultrasound transducer is a single-element transducer. In an embodiment, the ultrasound module comprises a high voltage generation module, a transceiver, a transceiver switch, and a microcontroller. The transceiver is in communication with the high voltage generation module to generate high-voltage excitation pulses based on control signals from the ultrasound transducer, where the generated high-voltage excitation pulses enable the ultrasound transducer to generate the characteristic waves based on the dynamic motion of the artery and one of the local and regional arterial stiffness of that artery. The transceiver switch is positioned between the ultrasound transducer and the transceiver. The microcontroller in communication with the transceiver switch and the transceiver, where the transceiver switch is operated based on a pulse control logic that is applied to the transceiver switch via the microcontroller, and where the pulse control logic is developed based on the high-voltage excitation pulses generated in the transceiver.

In an embodiment, the pulse detection module comprises an actuator control module and a pulse wave detector. The flow restrictor identifies the real-time pulse wave propagation pattern at the artery and generates the signals, and information regarding the signal is communicated to the actuator control module, and where the actuator control module transmits the information to the microcontroller. The pulse wave detector detects pulse wave patterns from the received signals from the flow restrictor, where the pulse wave detector transmits the detected pulse wave patterns to the microcontroller. In an embodiment, the microcontroller generates A-scan ultrasound frames based on the characteristic waves from the ultrasound module, where the A-scan ultrasound frames are transmitted to an automatic artery wall detection module of the measurement module, where the automatic artery wall detection module identifies wall boundaries of the artery. In an embodiment, the measurement module comprises a wall tracking module that traces the continuous movement of the identified wall boundaries of the arteries and produces a continuous motion pattern of the detected arterial wall boundaries.

In an embodiment, the measurement module comprises a diameter waveform generator module that receives the traced continuous movement of the arterial walls and waveforms from the wall tracking module to generate a diameter and distension waveform and characteristic waveforms linked to one of vibration and motion of the artery walls. In an embodiment, the measurement module further comprises a synchronized automatic cycle cutting and selection (SAC) module and a proximal diameter cycle module. The SAC module receives the diameter and distension waveform, where the SAC module extracts boundaries of the signal for individual cardiac cycles from the received waveforms, and where the SAC module further shares the signal boundaries to the proximal diameter cycle module. The proximal diameter cycle module measures signal magnitude and characteristics for individual cardiac cycles, and the local stiffness evaluation module generates the local stiffness indices.

In an embodiment, the measurement module comprises a beat-to-beat diameter generation module, a segregation module, a phasic diameter evaluation module, and an endothelial function module. The beat-to-beat diameter generation module is in communication with the diameter waveform generator module, where the diameter and distension waveform from the diameter waveform generator module is communicated to the beat-to-beat diameter generation module that measures diastolic diameter values from each cardiac beat. The segregation module is in communication with the beat-to-beat diameter generation module, where the segregation module receives the diastolic diameter values and diameter waveforms, and segregates diameter values for baseline state, low flow state, and vasodilation state. The phasic diameter evaluation module is in communication with the segregation module, where the phasic diameter evaluation module generates averaged baseline, peak dilated, and recovery diameter values from the segregated diameter values and waveforms received from the segregation module. The endothelial function module measures magnitude change and characteristics of averaged baseline, peak dilated, and recovery diameter received from the phasic diameter evaluation module, and assess the endothelial function.

In an embodiment, the measurement module further comprises a distal pulse wave module and a distal pulse cycle module. The distal pulse wave module receives the pulse wave generated by a pulse wave detector from the microcontroller, where the distal pulse wave module communicates the pulse wave to a processing module that processes the distal pulse wave. The processed distal pulse wave is communicated to the SAC module, where the SAC module extracts the pulse signal of individual cardiac cycles by synchronizing with the proximal diameter cycle module. The distal pulse cycle module in communication with the distal pulse wave module collates the extracted pulse signal from each cardiac beat, where the distal pulse cycle module in combination with the proximal diameter cycle module generates the regional stiffness indices. In an embodiment, the image-free ultrasound system simultaneously generates local stiffness indices, measures of endothelial function, and regional stiffness indices over continuous cardiac cycles, where generates values of individual cardiac cycles and the average.

### BRIEF DESCRIPTION OF DRAWINGS

The invention can be better understood with reference to the following drawings. The components in the drawings are not necessarily to scale, emphasis instead being placed upon clearly illustrating the principles of the present invention. Moreover, in the drawings, like reference numerals designate corresponding parts throughout the several views.
Figure 1 exemplarily illustrates a method flow diagram that shows a method for simultaneous and real-time non-invasive assessment of early vascular health markers using an image-free ultrasound system.
Figures 2A-2B exemplarily illustrate a schematic view of a hardware architecture of the image-free ultrasound system and software architecture that is associated with the hardware architecture, wherein the schematic view illustrates different steps involved in performing simultaneous and real-time non-invasive assessment of early vascular health markers using an image-free ultrasound system.

### DESCRIPTION OF THE INVENTION

The aim of the present invention is to provide an image-free ultrasound system for non-invasive assessment of early vascular health markers from superficial arteries by measurement of arterial dimensions and pulse waves to evaluate the endothelial function, local and regional vascular stiffness.

The foregoing advantages, as well as the particular construction of the image-free ultrasound system, will become more noticeable and understandable from the following detail description thereof when read in conjunction with the accompanying drawings.

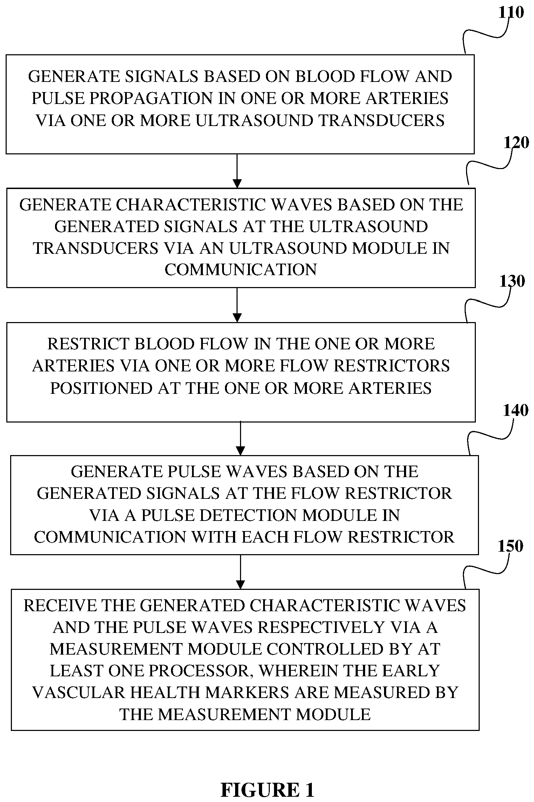

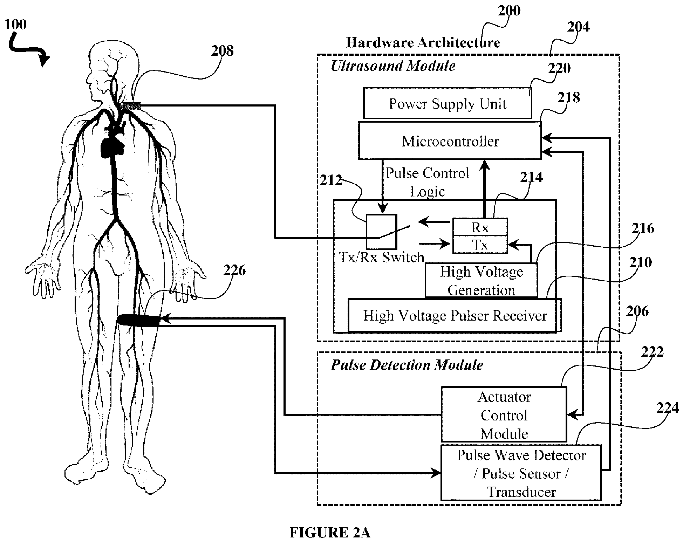

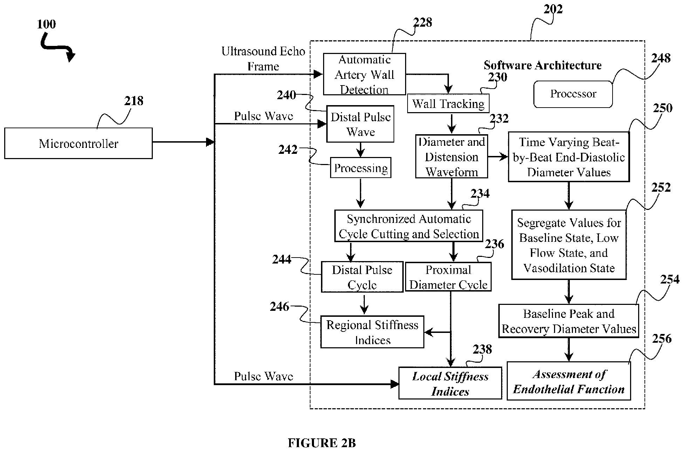

In general, based on Figures 1, 2A and 2B, the procedure involves simultaneous measurement of all the physiological parameters, wherein local stiffness measurements are obtained from the carotid artery using the single-element ultrasound probe, or in other words, the single-element ultrasound transducer, and the combination of the ultrasound probe (at the neck) and flow restrictor (at thigh) is used to measure regional stiffness from the carotid-femoral segment. The present disclosure describes a comprehensive image-free ultrasound system 100 for the assessment of vascular health with provisions for image-free, minimal operator dependency, portability, and field deployability. The image-free ultrasound system 100 performs simultaneous and real-time measurement of both the local and regional vascular stiffness and endothelial function, along with the arterial dimensions and their variations. The measurement sites for local stiffness include any superficial and large arteries. For example: common carotid arteries, femoral arteries, brachial arteries, aorta, etc. The measurable local stiffness indices include, but are not limited to: Arterial compliance, Young's modulus of elasticity, pressure-strain elastic modulus, arterial distensibility, stiffness index β, vessel wall rigidity coefficient, local PWV, systolic and diastolic PWV, change in PWV within a cardiac cycle, incremental PWV, local pulse transit time, wave reflection time, characteristic impedance, augmentation index, etc.

The measurement sites for regional stiffness include any arterial segment comprising of superficial, large, or peripheral arteries, for example, carotid-to-femoral, aorta-to-femoral, carotid-to-brachial, carotid-to-radial, brachial-to-radial, etc. The measurable regional stiffness indices include, but is not limited to aortic pulse wave velocity, pulse wave velocity, pulse transit time, aortic reflection transit time, etc. The image-free ultrasound system 100 measures arterial geometry and enables continuous capturing of high-fidelity waveforms of dynamic vascular geometry. The measurement sites for arterial dimensions include any superficial and large arteries, for example, common carotid arteries, femoral arteries, brachial arteries, aorta, etc. The measurable arterial dimensions include lumen diameter, instantaneous distension and diameter waveforms, intima-media thickness (wall thickness), instantaneous wall thickness waveform, displacement, velocity, and acceleration of arterial walls, etc.

The measurement sites for the assessment of endothelial function include any superficial and large arteries, which are safely occluded for sufficient duration, for example: brachial artery, radial artery, femoral artery, tibial artery, etc. The measure of endothelial function comprises multiple parameters that describe any form of abnormal activity or dysfunction of the endothelium. It also quantifies the ability of the target artery to dilate when stimulated with increasing shear stress.

In view of the above, the image-free ultrasound system 100 establishes the use of an image-free (or otherwise) single-element ultrasound probe along with a flow restrictor located over any superficial artery, along with intelligent algorithms, to perform simultaneous, continuous and real-time assessment of local and regional arterial stiffness and endothelial dysfunction (on a beat-to-beat basis).

The unique feature in image-free ultrasound system 100 is the synchronized and combined use of the custom flow restrictor 226 (to detect pulse waves from large peripheral arteries) with single-element ultrasound transducer 208 (to capture characteristic waves from the carotid artery). Such an arrangement, powered by fully automated measurement methods, enable real-time evaluation of the following measurements. The image-free ultrasound system 100 also captures characteristics motion of the carotid artery and pulse waves from large peripheral arteries, for example, femoral artery, to simultaneously evaluate local and regional stiffness, continuously from each cardiac cycle. Additionally, the system performs an FMD examination along with the stiffness measurement. From the measurement point of view, simultaneous and continuous assessment of all the relevant stiffness indices is a unique feature of the image-free ultrasound system 100 as disclosed here.

Figure 1 exemplarily illustrates a method flow diagram that shows a method for simultaneous and real-time non-invasive assessment of early vascular health markers using an image-free ultrasound system 100. Based on Figures 1-2B, the image-free ultrasound system 100 comprises one or more ultrasound transducers 208, an ultrasound module 204, one or more flow restrictors 226, a pulse detection module 206, and a measurement module 202. The ultrasound transducers 208 are positioned at one or more arteries, where the ultrasound transducers 208 generate 110 signals based on blood flow and pulse propagation in the arteries. The ultrasound transducer 208 is, for example, a single-element ultrasound transducer. The ultrasound module 204 is in communication with each ultrasound transducer 208, where the ultrasound module 204 generates 120 characteristic waves based on the generated signals at the ultrasound transducers 208. The flow restrictors 226 are positioned at the arteries to restrict 130 blood flow in the arteries, where the flow restrictors 226 generate signals based on the blood flow in the arteries.

The pulse detection module 206 is in communication with each flow restrictor 226, where the pulse detection module 206 generates 140 pulse waves based on the generated signals at the flow restrictor 226. The measurement module 202 or the software architecture as shown in Figure 2B, is controlled by at least one processor 248, where the measurement module 202 is in communication with the ultrasound module 204 and the pulse detection module 206 to receive 150 the generated characteristic waves and the pulse waves respectively, and where the early vascular health markers are measured by the measurement module 202 based on the characteristic waves and the pulse waves. In an embodiment, the early vascular health markers comprise regional stiffness indices, local stiffness indices, and assessment of endothelial function.

Figures 2A-2B exemplarily illustrate a schematic view of a hardware architecture 200 of the image-free ultrasound system 100 and software architecture or measurement module 202 that is associated with the hardware architecture, wherein the schematic view illustrates different steps involved in performing simultaneous and real-time non-invasive assessment of early vascular health markers using the image-free ultrasound system 100. As shown in Figure 2A and 2B, the ultrasound probe, or in other words, the ultrasound transducer 208 along with a flow restrictor (s) 226 which are located over a superficial artery. Based on Figure 2, the hardware architecture 200 includes an ultrasound module 204 and a pulse detection module 206, which are used to detect real-time assessment of local/regional arterial stiffness and endothelial function. In an embodiment, the ultrasound module 204 comprises a high voltage generation module 216, a transceiver 214, a transceiver (Tx/Rx) switch 212, and a microcontroller 218. The ultrasound module 204, which is powered by a power supply unit 220, includes the ultrasound transducer 208 that is in communication with a high voltage pulser receiver 210 via a transmitter/receiver (Tx/Rx) switch 212.

The transceiver 214 is in communication with the high voltage generation module 216 to generate high-voltage excitation pulses based on control signals from the ultrasound transducer 208, where the generated high-voltage excitation pulses enable the ultrasound transducer 208 to generate the characteristic waves based on the dynamic motion of the artery and one of the local and regional arterial stiffness of that artery. The transceiver Tx/Rx switch 212 is positioned between the ultrasound transducer 208 and the transceiver 214. The microcontroller 218 in communication with the transceiver Tx/Rx switch 212 and the transceiver 214, where the transceiver Tx/Rx switch 212 is operated based on a pulse control logic that is applied to the transceiver Tx/Rx switch 212 via the microcontroller 218. The pulse control logic is developed based on the high-voltage excitation pulses generated in the transceiver 214.

In other words, the Tx/Rx Switch 212 connects the ultrasound transducer 208 to a transceiver 214. The ultrasound transducer 208 is positioned on the superficial artery, as shown in Figure 1A and ultrasound is used to assess the local/regional arterial stiffness of that superficial artery. The high voltage generation module 216 works in combination with the transceiver 214 to generate high-voltage excitation pulses and receive a signal from the ultrasound transducer 208 that is associated with the dynamic motion of the artery/surrounding structure and local/regional arterial stiffness of that superficial artery. During the operation, the microcontroller 218 communicates with the transceiver 214 and Tx/Rx Switch 212. These modules 212 and 214 control the ultrasound transducer 208 while transmitting and receiving ultrasound signals, and the received ultrasound signal is fed to the microcontroller 218.

In an embodiment, the pulse detection module 206 comprises an actuator control module 222 and a pulse wave detector 224. The flow restrictor 226 identifies the real-time pulse wave propagation pattern at the artery and generates the signals, and information regarding the signal is communicated to the actuator control module 222. The actuator control module 222 transmits the information to the microcontroller 218. The pulse wave detector 224 detects pulse wave patterns from the received signals from the flow restrictor 226, and the pulse wave detector 224 transmits the detected pulse wave patterns to the microcontroller 218. In other words, the pulse detection module 206 includes the actuator control module 222 and the pulse wave detector/pulse sensor/transducer 224 (or the pulse wave detector 224). The flow restrictor 226 identifies the real-time pulse wave propagation pattern at the superficial artery, and the identified information is communicated to the actuator control module 222 and the pulse wave detector 224. The signals generated from both the actuator control module 222 and the pulse wave detector 224 are communicated to the microcontroller 218.

In an embodiment, the microcontroller 218 generates ultrasound echo frames based on the characteristic waves from the ultrasound module 204, where the ultrasound echo frames are transmitted to an automatic artery wall detection module 228 of the measurement module 202, where the automatic artery wall detection module 228 identifies wall boundaries of the artery. The microcontroller 218 communicates ultrasound echo frame frames and pulse wave information to the measurement module 202 that are associated with, for example, the local/regional arterial stiffness and endothelial function. In an embodiment, the measurement module 202 comprises a wall detection module 228 and a wall tracking module 230. The ultrasound echo frames are transmitted to an automatic artery wall detection module 228 that detects the wall of the superficial artery and the detected information is communicated to the wall tracking module 230 that traces the continuous movement of the identified wall boundaries of the arteries and produces continuous motion/vibration pattern of the detected arterial walls.

In the image-free ultrasound system 100 disclosed here, a single-element ultrasound transducer 208 (one unit of the ultrasound transmitter-receiver sensor) act as a source of sound energy. The ultrasound transducer 208 is configured to excite an arterial region such as a region of neck encompassing the carotid artery. Due to such excitation from a point source, sharp ultrasound echoes are generated from various structures present in the propagation path of the sound energy. These one-dimensional echo signals are captured with the same transducer and processed with an automated system to obtain a set of waveforms and measurements from the artery. There are no images or multi-dimensional illustrations are constructed using the captured ultrasound echoes to obtain the desired waveforms and measurements. Therefore, the ultrasound system 100 is entirely an image-free ultrasound system. On the hand, the disclosed system is unique from the conventional `ultrasound imaging systems' relay on the construction of ultrasound images from captured echoes to achieve any form of useful measurements. Further, an array of multiple sensors is typically required in such ultrasound imaging systems. By analyzing the one-dimensional echoes, the image-free ultrasound system 100 involves the processing of a set of characteristic waveforms from the artery. It can be any form of wall vibration or motion of the artery, for example, wall displacement, vessel's acceleration etc. Not necessarily only the distension or diameter waveform as described herein. Furthermore, any such characteristic waveform is used, along with suitable mathematical equations or physical models, to evaluate the local/regional stiffness indices and endothelial function.

In an embodiment, the measurement module 202 comprises a diameter waveform generator module 232 that receives the traced continuous movement of the arterial walls and waveforms from the wall tracking module 230 to generate a diameter and distension waveform, and characteristic waveforms linked to vibration or motion of the artery walls. The diameter waveform generator module 232 receives the tracked arterial wall motion pattern, waveforms from the wall tracking module 230, and generates a diameter and distension waveform, which is communicated to a synchronized automatic cycle cutting and selection module (SAC) 234. In an embodiment, the measurement module 202 further comprises the synchronized automatic cycle cutting and selection (SAC) module 234 and a proximal diameter cycle module 236. The SAC module 234 receives the diameter and distension waveform, where the SAC module 234 extracts the boundaries of the signal for individual cardiac cycles from the received waveform. The SAC module 234 further shares these signal boundaries to the proximal diameter cycle module 236 and the proximal diameter cycle module 236 measures signal magnitude and characteristics for individual cardiac cycle, and the local stiffness evaluation module 238 generates all the measures of local stiffness indices. In other words, the SAC module 234 shares the information of signal for each cardiac cycle to the proximal diameter cycle module 236, which then generates the local stiffness indices at the local stiffness evaluation module 238 using the extracted cycles.

In another embodiment, the measurement module 202 comprises a beat-to-beat diameter generation module 250, a segregation module 252, a phasic diameter evaluation module 254, and an endothelial function module 256. The beat-to-beat diameter generation module 250 is in communication with the diameter waveform generator module 232, where the diameter and distension waveform from the diameter waveform generator module 232 is communicated to the beat-to-beat diameter generation module 250 that measures diameter values (for instance, the end-diastolic diameter) from each cardiac beat. The segregation module 252 is in communication with the beat-to-beat diameter generation module 250, where the segregation module 252 receives the diameter values and segregates diameter values and diameter waveforms for three physiological phases, for example, baseline state, low flow state, and vasodilation state. The phasic diameter evaluation module 254 is in communication with the segregation module 252, where the phasic diameter evaluation module 254 generates averaged baseline, peak dilated, and recovery diameter values from the segregated diameter values or waveforms received from the segregation module 252. The endothelial function module 256 measures magnitude variation and characteristics of average baseline, peak dilated, and recovery diameter received from the phasic diameter evaluation module 254, and assess the endothelial function at the endothelial function module 256.

In other words, the assessment of endothelial function, the diameter and distension waveform generated from the diameter waveform generator module 232 is communicated to a beat-to-beat diameter generation module 250 that performs the evaluation of a time-varying beat by beat end-diastolic diameter values. The beat-to-beat diameter generation module 250 communicates the diastolic diameter values to segregation module 252 that segregates diameter values for baseline state, low flow state, and vasodilation state. The segregation module 252 communicates the segregated diameter values to the phasic diameter evaluation module 254 that generates averaged baseline, peak dilated and recovery diameter values. The generated average baseline, peak dilated, and recovery diameter values are communicated to the endothelial function module 256 that performs the assessment of endothelial function.

In an embodiment, the measurement module 202 further comprises a distal pulse wave module 240 and a distal pulse cycle module 244. The distal pulse wave module 240 receives the pulse wave generated by pulse wave detector 224 and from the microcontroller 218, and the distal pulse wave module 240 communicates the pulse wave to a processing module 242 that processes the distal pulse wave. The processed distal pulse wave is communicated to the SAC module 234 that extracts pulse signal of individual cardiac cycles by synchronizing with the proximal diameter cycle module 236. The distal pulse cycle module 244 in communication with the distal pulse wave module 240 collates the extracted pulse signal from each cardiac beat, and the distal pulse cycle module 244 in combination with the proximal diameter cycle module 236 generates the regional stiffness indices at module 246 using the extracted distal pulse cycles and proximal diameter cycles.

In view of the above, a pulse wave obtained from the pulse detection module 206 is transmitted to a distal pulse wave module 240. The distal pulse wave module 240 communicates the pulse wave to a processing module 242 that processes the distal pulse wave and communicates the processed digital pulse wave to the SAC module 234. The SAC module 234 communicates the signal to the module 246 that generates the regional stiffness indices. In another embodiment, the image-free ultrasound system 100 simultaneously generates local stiffness indices, measures of endothelial function, and regional stiffness indices over continuous cardiac cycles, where generates values of individual cardiac cycles and the average.

As will be appreciated by one of skill in the art, the present disclosure may be embodied as a method and system. Accordingly, the present disclosure may take the form of an entirely hardware embodiment, a software embodiment or an embodiment combining software and hardware aspects.

It will be understood that the functions of any of the units, as described above, can be implemented by computer program instructions. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts performed by any of the units as described above.

Instructions may also be stored in a computer-readable memory that can direct a computer or other programmable data processing apparatus to function in a particular manner, such that the instructions stored in the computer-readable memory produce an article of manufacture including instruction means which implement the function/act performed by any of the units as described above.

Instructions may also be loaded onto a computer or other programmable data processing apparatus like a scanner/check scanner to cause a series of operational steps to be performed on the computer or other programmable apparatus to produce a computer-implemented process such that the instructions which execute on the computer or other programmable apparatus provide steps for implementing the functions/acts performed by any of the units as described above.

## Claims

1. An image-free ultrasound system (100) for simultaneous, continuous and real-time non-invasive assessment of early vascular health markers comprising:
one or more ultrasound transducers (208) positioned at one or more arteries, wherein the ultrasound transducers (208) generate signals based on blood flow and pulse propagation in the arteries;
an ultrasound module (204) in communication with each ultrasound transducer (208), wherein the ultrasound module (204) generates characteristic waves based on the generated signals at the ultrasound transducers (208);
one or more flow restrictors (226) positioned at the one or more arteries to restrict blood flow in the one or more arteries, wherein the flow restrictors (226) generate signals based on the blood flow in the arteries;
a pulse detection module (206) in communication with each flow restrictor (226), wherein the pulse detection module (206) generates pulse waves based on the generated signals at the flow restrictor (226); and
a measurement module (202) controlled by at least one processor (248), wherein the measurement module (202) is in communication with the ultrasound module (204) and the pulse detection module (206) to receive the generated characteristic waves and the pulse waves respectively, wherein the early vascular health markers are measured by the measurement module (202) based on the characteristic waves and the pulse waves.

2. The image-free ultrasound system (100) as claimed in claim 1, wherein the early vascular health markers comprise regional stiffness indices, local stiffness indices, and assessment of endothelial function.

3. The image-free ultrasound system (100) as claimed in claim 1, wherein the ultrasound transducer (208) is a single-element ultrasound transducer (208).

4. The image-free ultrasound system (100) as claimed in claim 1, wherein the ultrasound module (204) comprises:
a high voltage generation module (216);
a transceiver (214) in communication with the high voltage generation module (216) to generate high-voltage excitation pulses based on control signals from the ultrasound transducer (208), wherein the generated high-voltage excitation pulses enable the ultrasound transducer (208) to generate the characteristic waves based on the dynamic motion of the artery and one of the local and regional arterial stiffness of that artery;
a transceiver switch (212) positioned between the ultrasound transducer (208) and the transceiver (214); and
a microcontroller (218) in communication with the transceiver switch (212) and the transceiver (214), wherein the transceiver switch (212) is operated based on a pulse control logic that is applied to the transceiver switch (212) via the microcontroller (218), wherein the pulse control logic is developed based on the high-voltage excitation pulses generated in the transceiver (214).

5. The image-free ultrasound system (100) as claimed in claim 1, wherein the pulse detection module (206) comprises:
an actuator control module (222), wherein the flow restrictor (226) identifies the real-time pulse wave propagation pattern at the artery and generates the signals, and information regarding the signal is communicated to the actuator control module (222), and wherein the actuator control module (222) transmits the information to the microcontroller (218); and
a pulse wave detector (224) detects pulse wave patterns from the received signals from the flow restrictor (226), wherein the pulse wave detector (224) transmits the detected pulse wave patterns to the microcontroller (218).

6. The image-free ultrasound system (100) as claimed in claim 1, wherein the microcontroller (218) generates ultrasound echo frames based on the characteristic waves from the ultrasound module (204), wherein the ultrasound echo frames are transmitted to an automatic artery wall detection module (228) of the measurement module (202), wherein the automatic artery wall detection module (228) identifies wall boundaries of the artery.

7. The image-free ultrasound system (100) as claimed in claim 1, wherein the measurement module (202) comprises a wall tracking module (230) that traces continuous movement of the identified wall boundaries of the arteries and produces continuous motion pattern of the detected arterial wall boundaries.

8. The image-free ultrasound system (100) as claimed in claim 7, wherein the measurement module (202) comprises a diameter waveform generator module that receives the traced continuous movement of the arterial walls and waveforms from the wall tracking module (230) to generate a diameter and distension waveform, and characteristic waveforms linked to one of vibration and motion of the artery walls.

9. The image-free ultrasound system (100) as claimed in claim 8, wherein the measurement module (202) further comprises:
a synchronized automatic cycle cutting and selection (SAC) module (234) that receives the diameter and distension waveform, wherein the SAC module (234) extracts boundaries of the signal for individual cardiac cycles from the received waveform, wherein the SAC module (234) further shares the signal boundaries to a proximal diameter cycle module (236);
the proximal diameter cycle module (236) measures signal magnitude and characteristics for individual cardiac cycles and
a local stiffness evaluation module that generates the local stiffness indices.

10. The image-free ultrasound system (100) as claimed in claim 9, wherein the measurement module (202) comprises:
a beat-to-beat diameter generation module in communication with the diameter waveform generator module, wherein the diameter and distension waveform from the diameter waveform generator module is communicated to the beat-to-beat diameter generation module that measures diastolic diameter values from each cardiac beat;
a segregation module in communication with the beat-to-beat diameter generation module, wherein the segregation module receives the diastolic diameter values and segregates diameter values for baseline state, low flow state, and vasodilation state;
a phasic diameter evaluation module in communication with the segregation module, wherein the phasic diameter evaluation module generates averaged baseline, peak dilated, and recovery diameter values from the segregated diameter values and waveforms received from the segregation module; and
an endothelial function module measures magnitude change and characteristics of the generated averaged baseline, peak dilated, and recovery diameter received from the phasic diameter evaluation module, and assess the endothelial function,

11. The image-free ultrasound system (100) as claimed in claim 9, wherein the measurement module (202) further comprises:
a distal pulse wave module that receives the pulse wave generated by pulse wave detector (224) from the microcontroller (218), wherein the distal pulse wave module communicates the pulse wave to a processing module that processes the distal pulse wave;
and communicates the processed digital pulse wave to the SAC module (234), wherein the SAC module (234) extracts pulse signal of individual cardiac cycles by synchronizing with the proximal diameter cycle module (236); and
a distal pulse cycle module (244) in communication with the distal pulse wave module (244) collates the extracted pulse signal from each cardiac beat, wherein the distal pulse cycle module (244) in combination with the proximal diameter cycle module (236) generates the regional stiffness indices.

12. The image-free ultrasound system (100) as claimed in claim 11 simultaneously generates local stiffness indices, measures of endothelial function, and regional stiffness indices over continuous cardiac cycles, wherein generates values of individual cardiac cycles and their average.

13. A method for simultaneous, continuous and real-time non-invasive assessment of early vascular health markers using an image-free ultrasound system (100), the method comprising:
generating signals based on blood flow and pulse propagation in one or more arteries via one or more ultrasound transducers (208) positioned at the one or more arteries;
generating characteristic waves based on the generated signals at the ultrasound transducers (208) via an ultrasound module (204) in communication with each ultrasound transducer (208);
restricting the blood flow in the one or more arteries via one or more flow restrictors (226) positioned at the one or more arteries, wherein the flow restrictors (226) generate signals based on the blood flow in the arteries;
generating pulse waves based on the generated signals at the flow restrictor (226) via a pulse detection module (206) in communication with each flow restrictor (226); and
receiving the generated characteristic waves and the pulse waves respectively via a measurement module (202) controlled by at least one processor (248), wherein the measurement module (202) is in communication with the ultrasound module (204) and the pulse detection module (206), and wherein the early vascular health markers are measured by the measurement module (202) based on the characteristic waves and the pulse waves.

## Patentansprüche

1. Bildloses Ultraschallsystem (100) zur gleichzeitigen, kontinuierlichen und nicht-invasiven Echtzeitbewertung früher Gefäßgesundheitsmarker, umfassend:
einen oder mehrere Ultraschallwandler (208), die an einer oder mehreren Arterien positioniert sind, wobei die Ultraschallwandler (208) Signale erzeugen, die auf Blutfluss und Pulsausbreitung in den Arterien basieren;
ein Ultraschallmodul (204), das mit jedem Ultraschallwandler (208) kommuniziert, wobei das Ultraschallmodul (204) charakteristische Wellen basierend auf den erzeugten Signalen an den Ultraschallwandlern (208) erzeugt;
einen oder mehrere Durchflussbegrenzer (226), die an der einen oder den mehreren Arterien positioniert sind, um den Blutfluss in der einen oder den mehreren Arterien zu beschränken, wobei die Durchflussbegrenzer (226) Signale basierend auf dem Blutfluss in den Arterien erzeugen;
ein Pulserkennungsmodul (206), das mit jedem Durchflussbegrenzer (226) kommuniziert, wobei das Pulserkennungsmodul (206) Pulswellen basierend auf den erzeugten Signalen an dem Durchflussbegrenzer (226) erzeugt; und
ein von mindestens einem Prozessor (248) gesteuertes Messmodul (202), wobei das Messmodul (202) mit dem Ultraschallmodul (204) und dem Pulserkennungsmodul (206) kommuniziert, um die erzeugten charakteristischen Wellen beziehungsweise die Pulswellen zu empfangen, wobei die frühen Gefäßgesundheitsmarker von dem Messmodul (202) basierend auf den charakteristischen Wellen und den Pulswellen gemessen werden.

2. Bildloses Ultraschallsystem (100) nach Anspruch 1, wobei die frühen Gefäßgesundheitsmarker regionale Steifigkeitsindizes, lokale Steifigkeitsindizes und eine Bewertung der Endothelfunktion umfassen.

3. Bildloses Ultraschallsystem (100) nach Anspruch 1, wobei der Ultraschallwandler (208) ein Einzelelement-Ultraschallwandler (208) ist.

4. Bildloses Ultraschallsystem (100) nach Anspruch 1, wobei das Ultraschallmodul (204) Folgendes umfasst:
ein Hochspannungserzeugungsmodul (216);
einen Sendeempfänger (214), der mit dem Hochspannungserzeugungsmodul (216) kommuniziert, um Hochspannungsanregungsimpulse basierend auf Steuersignalen vom Ultraschallwandler (208) zu erzeugen, wobei die erzeugten Hochspannungsanregungsimpulse dem Ultraschallwandler (208) ermöglichen, die charakteristischen Wellen basierend auf der dynamischen Bewegung der Arterie und einer von der lokalen und regionalen Arteriensteifigkeit dieser Arterie zu erzeugen;
einen Sendeempfängerschalter (212), der zwischen dem Ultraschallwandler (208) und dem Sendeempfänger (214) positioniert ist; und
einen Mikrocontroller (218), der mit dem Sendeempfängerschalter (212) und dem Sendeempfänger (214) kommuniziert, wobei der Sendeempfängerschalter (212) basierend auf einer Impulssteuerlogik betrieben wird, die über den Mikrocontroller (218) an den Sendeempfängerschalter (212) angelegt wird, wobei die Impulssteuerlogik basierend auf den im Sendeempfänger (214) erzeugten Hochspannungsanregungsimpulsen entwickelt wird.

5. Bildloses Ultraschallsystem (100) nach Anspruch 1, wobei das Pulserkennungsmodul (206) Folgendes umfasst:
ein Aktuatorsteuermodul (222), wobei der Durchflussbegrenzer (226) das Echtzeit-Pulswellenausbreitungsmuster an der Arterie identifiziert und die Signale erzeugt, und Informationen bezüglich des Signals an das Aktuatorsteuermodul (222) kommuniziert werden, und wobei das Aktuatorsteuermodul (222) die Informationen an den Mikrocontroller (218) überträgt; und
ein Pulswellendetektor (224) Pulswellenmuster aus den empfangenen Signalen vom Durchflussbegrenzer (226) erkennt, wobei der Pulswellendetektor (224) die erkannten Pulswellenmuster an den Mikrocontroller (218) überträgt.

6. Bildloses Ultraschallsystem (100) nach Anspruch 1, wobei der Mikrocontroller (218) Ultraschallechoeinzelbilder basierend auf den charakteristischen Wellen vom Ultraschallmodul (204) erzeugt, wobei die Ultraschallechoeinzelbilder an ein Modul zur automatischen Arterienwanderkennung (228) des Messmoduls (202) übertragen werden, wobei das Modul zur automatischen Arterienwanderkennung (228) Wandgrenzen der Arterie identifiziert.

7. Bildloses Ultraschallsystem (100) nach Anspruch 1, wobei das Messmodul (202) ein Wandverfolgungsmodul (230) umfasst, das eine kontinuierliche Bewegung der identifizierten Wandgrenzen der Arterien verfolgt und ein kontinuierliches Bewegungsmuster der erkannten Arterienwandgrenzen erzeugt.

8. Bildloses Ultraschallsystem (100) nach Anspruch 7, wobei das Messmodul (202) ein Durchmesserwellenform-Generatormodul umfasst, das die verfolgte kontinuierliche Bewegung der Arterienwände und Wellenformen vom Wandverfolgungsmodul (230) empfängt, um eine Durchmesser- und Dehnungswellenform sowie charakteristische Wellenformen, die mit einer von Vibration und Bewegung der Arterienwände verbunden sind, zu erzeugen.

9. Bildloses Ultraschallsystem (100) nach Anspruch 8, wobei das Messmodul (202) weiter Folgendes umfasst:
ein synchronisiertes automatisches Zyklusschneide- undauswahlmodul (SAC) (234), das die Durchmesser- und Dehnungswellenform empfängt, wobei das SAC-Modul (234) Grenzen des Signals für einzelne Herzzyklen aus der empfangenen Wellenform extrahiert, wobei das SAC-Modul (234) weiter die Signalgrenzen mit einem proximalen Durchmesserzyklusmodul (236) teilt;
das proximale Durchmesserzyklusmodul (236), das die Signalgröße und Eigenschaften für einzelne Herzzyklen misst, und
ein lokales Steifigkeitsauswertungsmodul, das die lokalen Steifigkeitsindizes erzeugt.

10. Bildloses Ultraschallsystem (100) nach Anspruch 9, wobei das Messmodul (202) Folgendes umfasst:
ein Schlag-zu-Schlag-Durchmesser-Generierungsmodul, das mit dem Durchmesserwellenform-Generatormodul kommuniziert, wobei die Durchmesser- und Dehnungswellenform vom Durchmesserwellenform-Generatormodul an das Schlag-zu-Schlag-Durchmesser-Generierungsmodul kommuniziert wird, das diastolische Durchmesserwerte von jedem Herzschlag misst;
ein Trennungsmodul, das mit dem Schlag-zu-Schlag-Durchmesser-Generierungsmodul kommuniziert, wobei das Trennungsmodul die diastolischen Durchmesserwerte empfängt und Durchmesserwerte für den Grundlinienzustand, den Zustand mit niedrigem Durchfluss und den Vasodilatationszustand trennt;
ein Modul zur Auswertung des phasischen Durchmessers, das mit dem Trennungsmodul kommuniziert, wobei das Modul zur Auswertung des phasischen Durchmessers gemittelte Grundlinien-, Spitzendilatations- und Erholungsdurchmesserwerte aus den vom Trennungsmodul empfangenen getrennten Durchmesserwerten und Wellenformen erzeugt; und
ein Endothelfunktionsmodul, das Größenänderung und Eigenschaften des erzeugten gemittelten Grundlinien-, Spitzendilatations- und Erholungsdurchmessers, die vom Modul zur Auswertung des phasischen Durchmessers empfangen werden, misst und die Endothelfunktion bewertet.

11. Bildloses Ultraschallsystem (100) nach Anspruch 9, wobei das Messmodul (202) weiter Folgendes umfasst:
ein distales Pulswellenmodul, das die vom Pulswellendetektor (224) erzeugte Pulswelle vom Mikrocontroller (218) empfängt, wobei das distale Pulswellenmodul die Pulswelle an ein Verarbeitungsmodul kommuniziert, das die distale Pulswelle verarbeitet;
und die verarbeitete digitale Pulswelle an das SAC-Modul (234) kommuniziert, wobei das SAC-Modul (234) Pulssignale einzelner Herzzyklen durch Synchronisieren mit dem proximalen Durchmesserzyklusmodul (236) extrahiert; und
ein distales Pulszyklusmodul (244), das in Kommunikation mit dem distalen Pulswellenmodul (244) das extrahierte Pulssignal von jedem Herzschlag erhebt, wobei das distale Pulszyklusmodul (244) in Kombination mit dem proximalen Durchmesserzyklusmodul (236) die regionalen Steifigkeitsindizes erzeugt.

12. Bildloses Ultraschallsystem (100) nach Anspruch 11, das gleichzeitig lokale Steifigkeitsindizes, Messungen der Endothelfunktion und regionale Steifigkeitsindizes über kontinuierliche Herzzyklen erzeugt, wobei es Werte einzelner Herzzyklen und deren Durchschnitt erzeugt.

13. Verfahren zur gleichzeitigen, kontinuierlichen und nicht-invasiven Echtzeitbewertung früher Gefäßgesundheitsmarker unter Verwendung eines bildlosen Ultraschallsystems (100), wobei das Verfahren Folgendes umfasst:
Erzeugen von Signalen basierend auf Blutfluss und Pulsausbreitung in einer oder mehreren Arterien über einen oder mehrere Ultraschallwandler (208), die an der einen oder mehreren Arterien positioniert sind;
Erzeugen charakteristischer Wellen basierend auf den erzeugten Signalen an den Ultraschallwandlern (208) über ein Ultraschallmodul (204), das mit jedem Ultraschallwandler (208) kommuniziert;
Beschränken des Blutflusses in der einen oder den mehreren Arterien über einen oder mehrere Durchflussbegrenzer (226), die an der einen oder den mehreren Arterien positioniert sind, wobei die Durchflussbegrenzer (226) Signale basierend auf dem Blutfluss in den Arterien erzeugen;
Erzeugen von Pulswellen basierend auf den erzeugten Signalen am Durchflussbegrenzer (226) über ein Pulserkennungsmodul (206), das mit jedem Durchflussbegrenzer (226) kommuniziert; und
Empfangen der erzeugten charakteristischen Wellen und der Pulswellen jeweils über ein von mindestens einem Prozessor (248) gesteuertes Messmodul (202), wobei das Messmodul (202) mit dem Ultraschallmodul (204) und dem Pulserkennungsmodul (206) kommuniziert und wobei die frühen Gefäßgesundheitsmarker von dem Messmodul (202) basierend auf den charakteristischen Wellen und den Pulswellen gemessen werden.

## Revendications

1. Système ultrasonore sans image (100) pour une évaluation non invasive simultanée, continue et en temps réel de marqueurs précoces de santé vasculaire comprenant :
un ou plusieurs transducteurs ultrasonores (208) positionnés au niveau d'une ou plusieurs artères, dans lequel les transducteurs ultrasonores (208) génèrent des signaux sur la base du débit sanguin et de la propagation d'impulsions dans les artères ;
un module ultrasonore (204) en communication avec chaque transducteur ultrasonore (208), dans lequel le module ultrasonore (204) génère des ondes caractéristiques sur la base des signaux générés au niveau des transducteurs ultrasonores (208) ;
un ou plusieurs limiteurs de débit (226) positionnés au niveau des une ou plusieurs artères pour limiter le débit sanguin dans les une ou plusieurs artères, dans lequel les limiteurs de débit (226) génèrent des signaux sur la base du débit sanguin dans les artères ;
un module de détection d'impulsions (206) en communication avec chaque limiteur de débit (226), dans lequel le module de détection d'impulsions (206) génère des ondes d'impulsion sur la base des signaux générés au niveau du limiteur de débit (226) ; et
un module de mesure (202) commandé par au moins un processeur (248), dans lequel le module de mesure (202) est en communication avec le module ultrasonore (204) et le module de détection d'impulsions (206) pour recevoir, respectivement, les ondes caractéristiques générées et les ondes d'impulsion, dans lequel les marqueurs précoces de santé vasculaire sont mesurés par le module de mesure (202) sur la base des ondes caractéristiques et des ondes d'impulsion.

2. Système ultrasonore sans image (100) selon la revendication 1, dans lequel les marqueurs précoces de santé vasculaire comprennent des indices de rigidité régionale, des indices de rigidité locale et une évaluation de la fonction endothéliale.

3. Système ultrasonore sans image (100) selon la revendication 1, dans lequel le transducteur ultrasonore (208) est un transducteur ultrasonore à élément unique (208).

4. Système ultrasonore sans image (100) selon la revendication 1, dans lequel le module ultrasonore (204) comprend :
un module de génération haute tension (216) ;
un émetteur-récepteur (214) en communication avec le module de génération haute tension (216) pour générer des impulsions d'excitation haute tension sur la base de signaux de commande provenant du transducteur ultrasonore (208), dans lequel les impulsions d'excitation haute tension générées permettent au transducteur ultrasonore (208) de générer les ondes caractéristiques sur la base du mouvement dynamique de l'artère et de l'une des rigidités artérielles locale et régionale de cette artère ;
un commutateur d'émetteur-récepteur (212) positionné entre le transducteur ultrasonore (208) et l'émetteur-récepteur (214) ; et
un microcontrôleur (218) en communication avec le commutateur d'émetteur-récepteur (212) et l'émetteur-récepteur (214), dans lequel le commutateur d'émetteur-récepteur (212) est actionné sur la base d'une logique de commande d'impulsions qui est appliquée au commutateur d'émetteur-récepteur (212) par l'intermédiaire du microcontrôleur (218), dans lequel la logique de commande d'impulsions est développée sur la base des impulsions d'excitation haute tension générées dans l'émetteur-récepteur (214).

5. Système ultrasonore sans image (100) selon la revendication 1, dans lequel le module de détection d'impulsions (206) comprend :
un module de commande d'actionneur (222), dans lequel le limiteur de débit (226) identifie le modèle de propagation d'ondes d'impulsion en temps réel au niveau de l'artère et génère les signaux, et des informations concernant le signal sont communiquées au module de commande d'actionneur (222), et dans lequel le module de commande d'actionneur (222) transmet les informations au microcontrôleur (218) ; et
un détecteur d'ondes d'impulsion (224) détecte des modèles d'ondes d'impulsion à partir des signaux reçus provenant du limiteur de débit (226), dans lequel le détecteur d'ondes d'impulsion (224) transmet les modèles d'ondes d'impulsion détectés au microcontrôleur (218).

6. Système ultrasonore sans image (100) selon la revendication 1, dans lequel le microcontrôleur (218) génère des trames échographiques ultrasonores sur la base des ondes caractéristiques du module ultrasonore (204), dans lequel les trames échographiques ultrasonores sont transmises à un module de détection automatique des parois artérielles (228) du module de mesure (202), dans lequel le module de détection automatique des parois artérielles (228) identifie des limites des parois de l'artère.

7. Système ultrasonore sans image (100) selon la revendication 1, dans lequel le module de mesure (202) comprend un module de suivi de parois (230) qui trace le mouvement continu des limites des parois identifiées des artères et produit un modèle de mouvement continu des limites de parois artérielles détectées.

8. Système ultrasonore sans image (100) selon la revendication 7, dans lequel le module de mesure (202) comprend un module générateur de formes d'ondes de diamètre qui reçoit le mouvement continu tracé des parois artérielles et des formes d'onde du module de suivi de parois (230) pour générer une forme d'onde de diamètre et de distension, et des formes d'onde caractéristiques liées à l'un parmi la vibration et le mouvement des parois artérielles.

9. Système ultrasonores sans image (100) selon la revendication 8, dans lequel le module de mesure (202) comprend en outre :
un module de coupe et de sélection de cycle automatique synchronisé (SAC) (234) qui reçoit la forme d'onde de diamètre et de distension, dans lequel le module SAC (234) extrait des limites du signal pour des cycles cardiaques individuels à partir de la forme d'onde reçue, dans lequel le module SAC (234) partage en outre les limites de signal avec un module de cycles de diamètre proximal (236) ;
le module de cycles de diamètre proximal (236) mesure l'amplitude et les caractéristiques de signal pour des cycles cardiaques individuels, et
un module d'évaluation de rigidité locale qui génère les indices de rigidité locale.

10. Système ultrasonore sans image (100) selon la revendication 9, dans lequel le module de mesure (202) comprend :
un module de génération de diamètre battement par battement en communication avec le module générateur de formes d'ondes de diamètre, dans lequel la forme d'onde de diamètre et de distension provenant du module générateur de formes d'ondes de diamètre est communiquée au module de génération de diamètre battement par battement qui mesure des valeurs de diamètre diastolique de chaque battement cardiaque ;
un module de séparation en communication avec le module de génération de diamètre battement par battement, dans lequel le module de séparation reçoit les valeurs de diamètre diastolique et sépare des valeurs de diamètre pour l'état initial, l'état de faible débit et l'état de vasodilatation ;
un module d'évaluation de diamètre phasique en communication avec le module de séparation, dans lequel le module d'évaluation de diamètre phasique génère des valeurs de diamètre initial moyen, de diamètre dilaté au maximum et de diamètre de récupération à partir des valeurs de diamètre séparées et de formes d'ondes reçues du module de séparation ; et
un module de fonction endothéliale mesure le changement d'amplitude et les caractéristiques du diamètre initial moyen, du diamètre dilaté au maximum et du diamètre de récupération générés reçus du module d'évaluation de diamètre phasique, et évalue la fonction endothéliale.

11. Système ultrasonore sans image (100) selon la revendication 9, dans lequel le module de mesure (202) comprend en outre :
un module d'ondes d'impulsion distale qui reçoit l'onde d'impulsion générée par le détecteur d'ondes d'impulsion (224) du microcontrôleur (218), dans lequel le module d'ondes d'impulsion distale communique l'onde d'impulsion à un module de traitement qui traite l'onde d'impulsion distale ;
et communique l'onde d'impulsion numérique traitée au module SAC (234), dans lequel le module SAC (234) extrait le signal d'impulsion de cycles cardiaques individuels par synchronisation avec le module de cycles de diamètre proximal (236) ; et
un module de cycles d'impulsion distal (244) en communication avec le module d'ondes d'impulsion distale (244) regroupe le signal d'impulsion extrait de chaque battement cardiaque, dans lequel le module de cycles d'impulsion distal (244) en combinaison avec le module de cycles de diamètre proximal (236) génère les indices de rigidité régionale.

12. Système ultrasonore sans image (100) selon la revendication 11, génère simultanément des indices de rigidité locale, des mesures de la fonction endothéliale et des indices de rigidité régionale sur des cycles cardiaques continus, dans lequel il génère des valeurs de cycles cardiaques individuels et leur moyenne.

13. Procédé d'évaluation non invasive simultanée, continue et en temps réel de marqueurs précoces de santé vasculaire en utilisant un système ultrasonore sans image (100), le procédé comprenant :
la génération de signaux sur la base du débit sanguin et de la propagation d'impulsions dans une ou plusieurs artères par l'intermédiaire d'un ou plusieurs transducteurs ultrasonores (208) positionnés au niveau des une ou plusieurs artères ;
la génération d'ondes caractéristiques sur la base des signaux générés au niveau des transducteurs ultrasonores (208) par l'intermédiaire d'un module ultrasonore (204) en communication avec chaque transducteur ultrasonore (208) ;
la limitation du débit sanguin dans les une ou plusieurs artères par l'intermédiaire d'un ou plusieurs limiteurs de débit (226) positionnés au niveau des une ou plusieurs artères, dans lequel les limiteurs de débit (226) génèrent des signaux sur la base du flux sanguin dans les artères ;
la génération d'ondes d'impulsion sur la base des signaux générés au niveau du limiteur de débit (226) par l'intermédiaire d'un module de détection d'impulsions (206) en communication avec chaque limiteur de débit (226) ; et
la réception des ondes caractéristiques générées et des ondes d'impulsion respectivement par l'intermédiaire d'un module de mesure (202) commandé par au moins un processeur (248), dans lequel le module de mesure (202) est en communication avec le module ultrasonore (204) et le module de détection d'impulsions (206), et dans lequel les marqueurs précoces de santé vasculaire sont mesurés par le module de mesure (202) sur la base des ondes caractéristiques et des ondes d'impulsion.
